(19) Europäisches Patentamt
European Patent Office
Office européen des brevets

(11) **EP 2 110 765 A1**

(12) **EUROPÄISCHE PATENTANMELDUNG**

(43) Veröffentlichungstag:
   **21.10.2009 Patentblatt 2009/43**

(51) Int Cl.:
   ***G06F 19/00*** *(2006.01)*

(21) Anmeldenummer: **09005546.8**

(22) Anmeldetag: **20.04.2009**

(84) Benannte Vertragsstaaten:
   **AT BE BG CH CY CZ DE DK EE ES FI FR GB GR HR HU IE IS IT LI LT LU LV MC MK MT NL NO PL PT RO SE SI SK TR**

(30) Priorität: **18.04.2008 DE 102008019627**

(71) Anmelder: **Samedi GmbH**
   **10115 Berlin (DE)**

(72) Erfinder:
   • **Keller, Katrin**
     **10119 Berlin (DE)**

   • **Alscher, Alexander Dr.**
     **10119 Berlin (DE)**
   • **Levin, Alexander**
     **13125 Berlin (DE)**

(74) Vertreter: **Lang, Johannes et al**
   **Bardehle, Pagenberg, Dost, Altenburg, Geissler**
   **Postfach 86 06 20**
   **81633 München (DE)**

(54) **Effizientes elektronisches Buchungssystem**

(57) Eine Vorrichtung zur elektronischen Buchung einer Dienstleistung, die umfasst: Mittel zur Speicherung von Informationen über Dienstleistungsarten, die einem Dienstleister zugeordnet sind, Mittel zur Speicherung von Informationen über Ressourcen, die dem Dienstleister zugeordnet sind, Mittel zur Speicherung von Informationen über die Verfügbarkeiten, die einer Ressource zugeordnet sind, Mittel zur Speicherung von Informationen über Buchungen, die einer Ressource zugeordnet sind, Mittel zur Speicherung einer Liste von Informationen über Dienstleistungsschritte, die einer Dienstleistungsart zugeordnet sind, wobei Informationen über einen Dienstleistungsschritt Informationen über mindestens eine Ressource und Informationen über einen Zeitraum umfasst, in dem die Ressource benötigt wird, eine Schnittstelle zum Erhalten einer Buchungsanfrage, die eine Dienstleistungsart spezifiziert, und Mittel zur Berechnung von Zeiten, zu denen die in der Buchungsanfrage spezifizierte Dienstleistungsart beim Dienstleister gebucht werden kann, wobei die Mittel dazu eingerichtet sind, die Berechnung anhand der der spezifizierten Dienstleistungsart zugeordneten Liste von Informationen über Dienstleistungsschritte, der Informationen über Ressourcen, die dem Dienstleister zugeordnet sind, der Informationen über die Verfügbarkeiten, die den Ressourcen zugeordnet sind und der Informationen über Buchungen, die den Ressourcen zugeordnet sind, durchzuführen.

Fig. 1

**Beschreibung**

**Gebiet der Erfindung**

Die vorliegende Erfindung betrifft ein System zur effizienten elektronischen Buchung einer Dienstleistung. Das System kann eine Buchungsanfrage empfangen, welche eine Dienstleistungsart spezifiziert, und auf effiziente Weise Zeiten ermitteln, zu denen die Dienstleistungsart bei einem Dienstleister wie zum Beispiel einer Arztpraxis gebucht werden kann.

An ein elektronisches Buchungssystem bestehen mannigfaltige Anforderungen: eine einfache Bedienbarkeit durch einen Kunden oder einen Dienstleister muss gewährleistet sein; bei der Buchung muss auf komplexe interne Abwicklungsmechanismen und Tätigkeitsabfolgen der beanspruchten Ressourcen (wie Arzt, Assistenz, Raum, Geräte, etc.) geachtet werden; die wirtschaftliche Auslastung der Ressourcen sollte optimiert werden; und eine rasche Systemantwort muss bereitgestellt sein, um die Akzeptanz beim Kunden und/oder beim Dienstleister sicherzustellen.

Vor allem die Schnelligkeit in der Generierung von Terminvorschlägen unter Berücksichtigung der zugrundeliegenden Tätigkeitsabfolgen, Buchungsregeln, Anfragepräferenzen und Optimierungsvorgaben stellt eine besondere technische Herausforderung dar. Die effiziente Generierung von Terminvorschlägen, d.h. die Antwortzeiten und der Ressourcenverbrauch (Rechenleistung/Speicherbedarf) eines die Terminanfrage bearbeitenden Systems, ist von entscheidender Bedeutung.

Zur Verdeutlichung sei exemplarisch eine große Klinik mit über 30 Ressourcen und 200 Dienstleistungsangeboten genannt. Ein Dienstleistungsangebot kann dabei mehrere Ressourcen beanspruchen, bspw. bei einem Operationstermin anfangs die Anästhesie 15 Minuten, anschließend den Arzt, Assistenz und den Laser 30 Minuten, abschließlich die Anästhesie-Assistenz 15 Minuten und während der ganzen Zeit OP-Raum 1 oder 3 (aber nicht OP-Raum 2). Falls ein überweisender Arzt oder ein Patient diese Dienstleistung OP-Termin buchen möchte, so muss garantiert sein, dass die oben genannten sechs Ressourcen noch verfügbar sind. Bei solch einer Klinik mit über 100.000 Terminen (bzw. Patienten) pro Jahr, kann sich die Schnittmenge an verfügbaren Ressourcen und damit das Terminangebot innerhalb von Sekunden verändern.

**Stand der Technik**

Anordnungen und Verfahren zur Buchung von Terminen und Dienstleistungen beispielsweise für eine Flugbuchung sind bekannt und verbreitet. Allerdings beruht beispielsweise eine Flugbuchung auf einem starren Flugplan. Terminplaner erfordern eine Kenntnisnahme einer Terminanforderung durch den Empfänger.

Bei einem Flugbuchungssystem werden zur Buchung einer Flugreise vom Ort A zum Ort B zunächst eine Strecke und ein Datum gewählt. Das Flugbuchungssystem zeigt dann ein Leistungsangebot (Verfügbarkeit und Preis) an, woraufhin der Nutzer eine Leistung auswählt. Allerdings ist die Terminbuchung nur aufgrund eines fest vorgegebenen, starren Flugplans möglich. Nachteilig an einem derartigen Flugbuchungssystem ist, dass eine Optimierung, wenn überhaupt, nur bezüglich der Strecke möglich ist.

Ferner sind für medizinische Dienstleistungen Systeme zur Online-Terminvereinbarung bei einem Arzt bekannt. Ein Patient wählt hierbei einen Arzt, eine Leistung und ein Datum aus und lässt sich ein Uhrzeit-Angebot für eine Konsultation anzeigen. Daraufhin wählt der Patient eine Uhrzeit aus und nimmt eine Buchung vor. Falls ein medizinischer Leistungserbringer einen reinen Online-Kalender führt, so beinhaltet der Online-Terminkalender meistens nur Standard-Termine mit vorgegebenen fixen Längen, ohne dass die individuellen praxisinternen Ressourcen oder Besonderheiten einzelner Praxen berücksichtigt werden. Falls mehrere Ressourcen und Tätigkeitsabfolgen berücksichtigt werden, sind die Online-Kalender sehr langsam in den Antwortzeiten aufgrund der Auslastung der Rechenkapazität bei der jeweils einzeln erfolgenden Ressourcenprüfung.

**Zusammenfassung der Erfindung**

Eine Aufgabe der vorliegenden Erfindung ist es daher, ein System zur elektronischen Buchung zur Verfügung zu stellen, bei dem Dienstleistungsangebote möglichst gut mit den Gegebenheiten bei einem Dienstleister abgestimmt werden können und das weiterhin eine effiziente Berechnung verfügbarer Termine und damit schnelle und einfache Dienstleistungsbuchungen ermöglicht.

Diese Aufgabe wird durch die Merkmale des Anspruches 1 gelöst. Vorteilhafte Ausführungsformen ergeben sich aus den abhängigen Ansprüchen.

Eine erfindungsgemäße Vorrichtung zur elektronischen Buchung einer Dienstleistung, umfasst Mittel zur Speicherung von Informationen über Dienstleistungsarten, die einem Dienstleister zugeordnet sind, Mittel zur Speicherung von Informationen über Ressourcen, die dem Dienstleister zugeordnet sind, Mittel zur Speicherung von Informationen über die Verfügbarkeiten, die einer Ressource zugeordnet sind, Mittel zur Speicherung einer Liste von Informationen über Buchungen, die einer Ressource zugeordnet sind, und Mittel zur Speicherung einer Liste von Informationen über Dienstleistungsschritte, die einer Dienstleistungsart zugeordnet sind, wobei Informationen über einen Dienstleistungsschritt Informationen über mindestens eine Ressource und Informationen über einen Zeitraum umfasst, in dem die Ressource benötigt wird. Die Vorrichtung umfasst weiterhin eine Schnittstelle zum Erhalten einer Buchungsanfrage, die eine Dienstleistungsart spezifiziert. Schließlich umfasst die Vorrichtung Mittel zur Berechnung von Zeiten, zu denen die in der Buchungsanfrage spezifizierte Dienstleistungsart beim Dienstleister gebucht werden kann, wobei die Mittel dazu eingerichtet sind, die Berechnung anhand der der spezifizierten Dienstleistungsart zugeordneten Liste von Informationen über Dienstleistungsschritte, der Informationen über Ressourcen, die dem Dienstleister zugeordnet sind, der Informationen über die Verfügbarkeiten, die den Ressourcen zugeordnet sind und der Informationen über Buchungen, die den Ressourcen zugeordnet sind, durchzuführen..

Mit der vorliegenden Erfindung werden mit Hilfe der oben erwähnten Mittel zur Speicherung und Mittel zur Berechnung sehr effiziente Berechnung freier Termine trotz komplexer Dienstleistungsangebote ermöglicht. Darüber hinaus bietet die vorliegende Erfindung einem Dienstleister die Möglichkeit, nicht nur den Terminkalender sondern auch die internen Abläufe individuell selbst zu konfigurieren. Dadurch können komplexe interne Abläufe eines Dienstleisters auf der Grundlage konfigurierter Ressourcen mit einem Terminkalender unter Optimierung abgeglichen werden.

Bei einer Buchung werden folgende Schritte ausgeführt: Empfangen einer Dienstleistungs-Buchungsanforderung und Ermitteln freier Termine auf der Grundlage der konfigurierten Ressourcen des Dienstleisters in Verbindung mit dem Terminkalender des Dienstleisters. Beispielsweise wählen Kunden einen Dienstleister, Terminarten bzw. Leistungen die vom Dienstleister angeboten werden und ein Datum. Anschließend können dem Kunden freie Termine mit Uhrzeiten angezeigt werden, die anhand der Konfiguration und dem zugrunde liegenden Buchungsalgorithmus des Dienstleisters ermittelt wurden. Die Termine können dann zum Beispiel durch Anklicken der Uhrzeit vom Kunden gebucht werden. Der Nutzer kann vorzugsweise ein Kunde sein, der mit einer Kundenberechtigung unter Verwendung seines Kunden-Rechners Zugang zur erfindungsgemäßen Anordnung erhält, aber natürlich ist der Dienstleister auch in der Lage, seinen eigenen Terminkalender auch selbst zu verwalten. In diesem Falle tritt der Dienstleister vorzugsweise als Nutzer mit einer Dienstleisterberechtigung auf und nimmt die Terminbuchung über den Dienstleister-Rechner vor.

Die oben erwähnten Listen von Informationen können zum Beispiel als verkettete Listen, als Datenfelder (Arrays) oder in einer beliebigen anderen Form implementiert werden.

Die Informationen über Dienstleistungsschritte können Informationen über mindestens eine Ressource durch einen direkten Verweis auf die mindestens eine Ressource umfassen oder dadurch, dass die Informationen über die mindestens eine Ressource Teil der Datenstruktur der Informationen über Dienstleistungsschritte sind. Informationen über Dienstleistungsschritte können Informationen über mindestens eine Ressource aber auch indirekt umfassen. So umfasst in einer bevorzugten Ausführungsform der Erfindung die Vorrichtung Mittel zur Speicherung einer Liste von Informationen über Fähigkeiten, die einer Ressource zugeordnet sind. Informationen über einen Dienstleistungsschritt umfassen die Informationen über mindestens eine Ressource als Informationen über eine benötigte Fähigkeit. Die Mittel zur Berechnung von Zeiten, zu denen die in der Buchungsanfrage spezifizierte Dienstleistungsart beim Dienstleister gebucht werden kann, sind dazu eingerichtet, anhand der Informationen über die benötigte Fähigkeit Informationen über Ressourcen zu ermitteln, denen die benötigte Fähigkeit zugeordnet ist.

Dadurch wird die interne Optimierung von Ressourcen (z.B. Ärzte, Assistenten, Räume oder Geräte) und deren - gegebenenfalls austauschbaren - Fähigkeiten berücksichtigt. Dies stellt gegenüber den eingangs beispielsweise erwähnten herkömmlichen Buchungssystemen dadurch eine Verbesserung dar, dass eine Optimierung von zwei unabhängigen Dimensionen, Ressource und Fähigkeiten, bezogen auf die Dienstleistungsart vorgenommen wird. Die Zeiten können dynamisch nach einem Buchungsalgorithmus ermittelt und auf die Verfügbarkeiten von Ressourcen, Fähigkeiten und Dienstleistungsarten überprüft werden.

In einer weiteren bevorzugten Ausführungsform der Erfindung umfassen die Informationen über die Verfügbarkeiten einen Bitstring, in dem jedes Bit die Verfügbarkeit in einem bestimmten Zeitintervall beschreibt. Weiterhin umfassen die Informationen über Buchungen einen Bitstring, aus dem ermittelt werden kann, zu welchem Zeitpunkt gebucht oder nicht gebucht ist. Schließlich sind die Mittel zur Berechnung von Zeiten, zu denen die in der Buchungsanfrage spezifizierte Dienstleistungsart beim Dienstleister gebucht werden kann, dazu eingerichtet, für einen gegebenen Zeitraum die Zeiten dadurch zu berechnen, dass: die der spezifizierten Dienstleistungsart zugeordneten Dienstleistungsschritte ermittelt werden; für die ermittelten Dienstleistungsschritte jeweils mindestens eine Ressource ermittelt wird; für die ermittelten Ressourcen die Verfügbarkeiten und die Buchungen ermittelt werden, die der jeweiligen Ressource zugeordnet sind; die Bitstrings der ermittelten Verfügbarkeiten, und Buchungen durch mindestens eine erste Operation so verknüpft werden, dass jeweils ein Bitstring erzeugt wird, der die Buchbarkeit der jeweiligen Ressource im gegebenen Zeitraum beschreibt; aus der Buchbarkeit der für einen Dienstleistungsschritt ermittelten Ressourcen ein Bitstring ermittelt wird, der die Buchbarkeit des jeweiligen Dienstleistungsschritts im gegebenen Zeitraum beschreibt; die ermittelten Bitstrings der Buchbarkeiten der Dienstleistungsschritte durch mindestens eine zweite Operation so verknüpft werden, dass ein Bitstring erzeugt wird, der die Buchbarkeit der spezifizierten Dienstleistungsart im gegebenen Zeitraum beschreibt.

Mit dieser Ausführungsform der Erfindung wird die elektronische Suche nach möglichen Terminen für eine Dienstleistung besonderes effizient umgesetzt. Eine optimierte elektronische Ermittlung eines freien Termins erfolgt auf der Grundlage einer Repräsentation des Terminkalenders in Form von Bitfeldern, wobei zur Ermittlung freier Termine eine logische Verknüpfung der Terminkalender-Bitfelder mit anderen vorbestimmten Bitfeldern erfolgt. Hierdurch ist die Optimierung besonders schnell im Vergleich zu herkömmlichen Verfahren, die beispielsweise einen Feld-für-Feld-Vergleich durchführe. Dabei werden die Zeitpunkte zu denen eine Ressource eines Dienstleisters nicht gebucht ist, als eine Folge von Bits in der Weise gespeichert, dass der Zustand jedes Bits markiert, ob eine Ressource in einem Intervall (beispielsweise 1 Minute) buchbar ist. Der Index jedes Bits gibt an, welcher absolute Zeitpunkt des jeweiligen Bits beschrieben wird. Die Verfügbarkeiten (Öffnungszeiten) jeder Ressource werden analog codiert, jedoch mit dem Unterschied, dass hier der Index in den Bitstring über Kongruenzarithmetik bezüglich eines beliebigen aber festen Moduls (etwa eine Woche) aus einem gegebenen Zeitpunkt berechnet wird, da Öffnungszeiten sich wiederholen. Die Schritte zur Berechnung der Buchbarkeit von Ressourcen, Dienstleistungsschritten und Dienstleistungsarten sind hauptsächlich bitweise logische Verknüpfungen auf Ganzzahlen oder ähnliche elementare mathematische Operationen, die sehr effizient auf Computersystemen ausführbar sind. Dadurch zeichnet sich der in dieser Erfindung beschriebene Ablauf durch hohe Geschwindigkeit für typische Abfragen aus.

In einer weiteren bevorzugten Ausführungsform der Erfindung sind die Mittel zur Berechnung von Zeiten, zu denen die in der Buchungsanfrage spezifizierte Dienstleistungsart beim Dienstleister gebucht werden kann, dazu eingerichtet, zur Ermittlung des Bitstrings, der die Buchbarkeit eines Dienstleistungsschritts im gegebenen Zeitraum beschreibt, die erzeugten Bitstrings der Buchbarkeiten der für den Dienstleistungsschritt ermittelten Ressourcen durch mindestens eine dritte Operation so zu verknüpfen, dass der zu ermittelnde Bitstring erzeugt wird.

In einer weiteren bevorzugten Ausführungsform der Erfindung weist die Vorrichtung eine Schnittstelle auf, über die Buchungsvorgaben in Form einer Basiskonfiguration des Dienstleisters empfangen werden können.

Die Vorrichtung ist dazu hergerichtet, auf Empfang einer Dienstleistungs-Buchungsanforderung auf der Grundlage der gespeicherten Basiskonfiguration des Dienstleisters in Verbindung mit dem Terminkalender des Dienstleisters freie Termine für die angeforderte Dienstleistung zu ermitteln. Hierbei fungiert die Basiskonfiguration als eine Art Steuertabelle für die Terminermittlung. Die Basiskonfiguration kann zum Beispiel Informationen über die Dienstleistungsarten enthalten, die von einem Dienstleister angeboten werden, Informationen über die Dienstleistungsschritte einer Dienstleistungsart, Informationen über Fähigkeiten, die für einen Dienstleistungsschritt benötigt werden, Informationen über Fähigkeiten, über die Ressourcen des Dienstleisters verfügen, und Informationen über die Verfügbarkeit von Ressourcen. In dem Fall, dass die Vorrichtung zur elektronischen Buchung ein Server ist, kann die Basiskonfiguration eines Dienstleisters an den Server durch einen Client des Dienstleisters übertragen werden. Der Server kann die Basiskonfiguration des Dienstleisters empfangen und speichern und die Tätigkeitsabläufe und Buchungsregeln des Dienstleisters nach Maßgabe der Basiskonfiguration verwalten und auf Empfang einer Dienstleistungs-Buchungsanforderung von einem Client-Rechner auf der Grundlage der gespeicherten Basiskonfiguration des Dienstleisters in Verbindung mit dem Terminkalender des Dienstleisters freie Termine für die angeforderte Dienstleistung ermitteln und dem Client-Rechner zur Anzeige zu übertragen.

In einer weiteren bevorzugten Ausführungsform der Erfindung weist die Vorrichtung eine Interaktionseinheit auf, die dazu eingerichtet ist, auf eine Buchungsanfrage hin eine Mehrzahl von Dienstleistern anzuzeigen, die die angefragte Dienstleistungsart bereitstellen.

Beispielsweise wird in dieser bevorzugten Ausführungsform eine Mehrzahl von Dienstleistern auf einem Server unterstützt. Dadurch kann der Nutzer auf einfache Weise nur seine gewünschte Dienstleistung angeben und erhält alle Dienstleister, die diese Dienstleistung anbieten. Der Nutzer wählt sich dann beispielsweise den nächstgelegenen Dienstleister aus und führt dann seine Terminbuchung durch. Auf diese Weise wird beispielsweise den Nutzern in einer Stadt eine flächendeckende Versorgung hinsichtlich einer gewünschten Dienstleistung angeboten, während es den Dienstleistern erleichtert ist, ihre Ressourcen auszulasten.

In einer weiteren bevorzugten Ausführungsform der Erfindung ist die Interaktionseinheit dazu eingerichtet, die angezeigten Dienstleister nach ihrer räumlichen Entfernung zu einem Nutzer sortiert anzuzeigen.

Beispielsweise ist in dieser bevorzugten Ausführungsform ein Server dazu eingerichtet, die Mehrzahl von Dienstleistern sortiert nach einem räumlichen Abstand vom Kunden anzuzeigen. Beispielsweise wird der dem Kunden nächstgelegene Dienstleister auf der Liste zuoberst angezeigt. Alternativ kann auch vorgesehen sein, dass nur der nächstgelegene Dienstleister angezeigt wird. Der Nutzer kann dann den ihm nächstgelegenen Dienstleister in einfacher Weise ermitteln und in dessen Terminkalender eine Buchung vornehmen.

In einer weiteren bevorzugten Ausführungsform der Erfindung ist die Interaktionseinheit dazu eingerichtet, die angezeigten Dienstleister nach der Dauer bis zum frühest möglichen Zeitpunkt der Erbringung der Dienstleistungsart sortiert anzuzeigen.

Beispielsweise ist in dieser bevorzugten Ausführungsform ein Server dazu eingerichtet, die Mehrzahl von Dienstleistern sortiert nach ihrer zeitlichen Verfügbarkeit anzuzeigen. Beispielsweise wird derjenige Dienstleister zuoberst angezeigt, der als erstes einen freien Termin anbietet.

Dem Nutzer kann bei Vorgabe einer gewünschten Dienstleistung auch eine Liste von Dienstleistern mit freien Terminen angeboten werden, die sowohl räumlich als auch zeitlich nächstgelegen sind. Dadurch wird ein dreidimensionaler Abgleich hinsichtlich Leistung, Raum und Zeit und/oder eine Koordination von Bedürfnissen von Kunden (beispielsweise Konsumenten, Nachfrager oder Patienten) und den von den Dienstleistern (beispielsweise Anbieter oder Ärzte) angebotenen Leistungen bereitgestellt. Dabei werden gemäß den Leistungs-, Raum- und Zeitpräferenzen des Kunden, dem Kunden ein optimales Angebot von Leistungs-, Raum- und Zeitverfügbarkeiten von mehreren Anbietern unterbreitet.

Ein erfindungsgemäßes Verfahren zur elektronischen Buchung einer Dienstleistung, umfasst Speichern von Informationen über Dienstleistungsarten, die einem Dienstleister zugeordnet sind; Speichern von Informationen über Ressourcen, die dem Dienstleister zugeordnet sind; Speichern von Informationen über die Verfügbarkeiten, die einer Ressource zugeordnet sind; Speichern von Informationen über Buchungen, die einer Ressource zugeordnet sind; Speichern einer Liste von Informationen über Dienstleistungsschritte, die einer Dienstleistungsart zugeordnet sind, wobei Informationen über einen Dienstleistungsschritt Informationen über mindestens eine Ressource und Informationen über einen Zeitraum umfasst, in dem die Ressource benötigt wird; Erhalten einer Buchungsanfrage, die eine Dienstleistungsart spezifiziert; und Berechnen von Zeiten, zu denen die in der Buchungsanfrage spezifizierte Dienstleistungsart beim Dienstleister gebucht werden kann, anhand der der spezifizierten Dienstleistungsart zugeordneten Liste von Informationen über Dienstleistungsschritte, der Informationen über Ressourcen, die dem Dienstleister zugeordnet sind, der Informationen über die Verfügbarkeiten, die den Ressourcen zugeordnet sind und der Informationen über Buchungen, die den Ressourcen zugeordnet sind.

Die Schritte des erfindungsgemäßen Verfahrens brauchen nicht notwendigerweise in der Reihenfolge ausgeführt werden, in der sie oben aufgezählt sind.

Die Erfindung kann als ein Computerprogramm-Produkt mit Programmcode ausgeführt sein, der bewirkt, dass das beschriebene Verfahren zur elektronischen Buchung einer Dienstleistung bei einem Dienstleister durch einen Nutzer durchgeführt wird, wenn der Programmcode auf einem Computer ausgeführt wird. Beispielsweise kann das erfindungsgemäße Computerprogramm über das Internet geladen und installiert werden. Alternativ kann das Computerprogramm in auf einem Datenträger gespeicherter Form bereit gestellt werden. Beispielsweise kann der Datenträger als CD, DVD, USB-Stick oder anderweitiger tragbarer Datenträger ausgeführt sein.

**Kurze Beschreibung der Zeichnungen**

In den Zeichnungen zeigt

Fig. 1    Arten von Informationen, die in einer Ausführungsform der Erfindung durch das Buchungssystem gespeichert werden;

Fig. 2a    eine beispielhafte Datenstruktur einer Dienstleistungsart;

Fig. 2b    eine graphische Darstellung der Dienstleistungsart aus Figur 2b;

Fig. 3    die Berechnung der Buchbarkeit einer Ressource, gemäß einer Ausfüh- rungsform der Erfindung;

Fig. 4    die Berechnung der Buchbarkeit eines Dienstleistungsschritts, gemäß einer Ausführungsform der Erfindung;

Fig. 5    die Berechnung der Buchbarkeit einer Dienstleistungsart, gemäß einer Ausführungsform der Erfindung;

Fig. 6    eine schematische Darstellung der Struktur einer Internet-gestützten Aus- führungsform der Erfindung;

Fig. 7    ein Beispiel eines Konfigurationsmenüs zur Konfiguration der Ausfüh- rungsform der Erfindung nach Fig. 6;

Fig. 8    ein Beispiel einer Terminkalenderansicht aus der Sicht des Dienstleisters gemäß einer Ausführungsform der Erfindung; und

Fig. 9    ein Beispiel einer Kunden-Online-Terminvergabe und Sicht des Terminka- lenders beim Dienstleister gemäß einer Ausführungsform der Erfindung.

**Ausführliche Beschreibung bevorzugter Ausführungsformen**

Im Folgenden werden Ausführungsformen der Erfindung beispielhaft beschrieben.

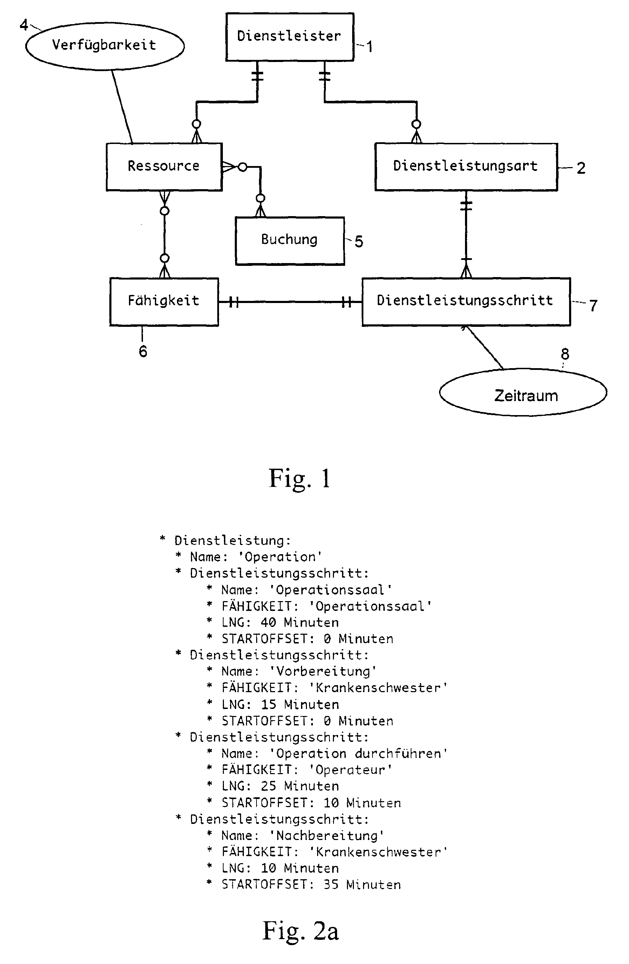

Figur 1 zeigt die Arten von Informationen, die in einer Ausführungsform der Erfindung durch das Buchungssystem gespeichert werden. Dabei sind einem Dienstleister eine Mehrzahl von Information über Dienstleistungsarten und eine Mehrzahl von Informationen über Ressourcen zugeordnet. Jeder Ressource sind Informationen über ihre allgemeine Verfügbarkeit (Öffnungszeit) und Informationen über ihre Buchung zugeordnet. In einer alternativen Ausführungsform (nicht dargestellt) ist einer Ressource eine Mehrzahl von Informationen über Buchungen zugeordnet. Weiterhin sind jeder Ressource auch Informationen über die Fähigkeiten der Ressource zugeordnet, die die Ressource besitzt. Informationen über eine Dienstleistungsart sind eine Mehrzahl Informationen über Dienstleistungsschritte zugeordnet. Den Informationen über einen Dienstleistungsschritt sind Informationen über eine Fähigkeit zugeordnet, welche für den Dienstleistungsschritt benötigt wird, und Informationen über einen Zeitraum, in dem die Fähigkeit benötigt wird. Anhand der oben erwähnten Zuordnung der Ressourcen zu Fähigkeiten kann das Buchungssystem somit eine oder mehrere Ressourcen ermitteln, welche die im Dienstleistungsschritt benötigte Fähigkeit besitzen. In einer alternativen Ausführungsform (nicht dargestellt) sind ist einem Dienstleistungsschritt mindestens eine Ressource direkt zugeordnet.

Die Figur 2a zeigt eine beispielhafte Datenstruktur einer Dienstleistungsart. Dabei umfasst die Dienstleistungsart eine Liste von Dienstleistungsschritten, sowie optionale weitere Daten, etwa den Namen der Dienstleistungsart. In der Figur 2a wird eine Dienstleistungsart mit dem Namen "Operation" und vier Dienstleistungsschritten gezeigt. Jeder Dienstleistungsschritt in der Liste von Dienstleistungsschritten umfasst in der Figur 2a Informationen über eine Fähigkeit, die in dem Dienstleistungsschritt benötigt werden, Informationen über die Dauer (Länge LNG) des Dienstleistungsschritt, Informationen über die Zeitversetzung (STARTOFFSET) gegenüber dem Beginn der gesamten Dienstleistung, sowie optionale weitere Daten, etwa den Namen des Dienstleistungsschritts. So umfasst der Eintrag in der Liste von Dienstleistungsschritten in der Figur 2a die Informationen, dass in einem Dienstleistungsschritt mit dem Namen "Operation durchführen" eine Fähigkeit "Operateur" 25 Minuten lang benötigt wird, beginnend 10 Minuten nach dem Beginn der gesamten Dienstleistung. Über die Information über die Fähigkeit, die in dem Dienstleistungsschritt benötigt werden, lassen sich eine oder mehrere Ressourcen des Dienstleisters ermitteln, die mit der Fähigkeit assoziiert sind, d.h. die diese Fähigkeit besitzen. Alternativ könnten die Informationen über den Dienstleistungsschritt auch direkt Informationen über eine oder mehrere alternative Ressourcen umfassen oder auf diese verweisen. Die Informationen über die Dauer (LNG) und die Zeitversetzung (STARTOFFSET) bilden die oben erwähnten Informationen über den Zeitraum, in dem die Ressource benötigt wird, welche die Informationen über den Dienstleistungsschritt direkt oder indirekt (z.B. über eine Fähigkeit) umfassen.

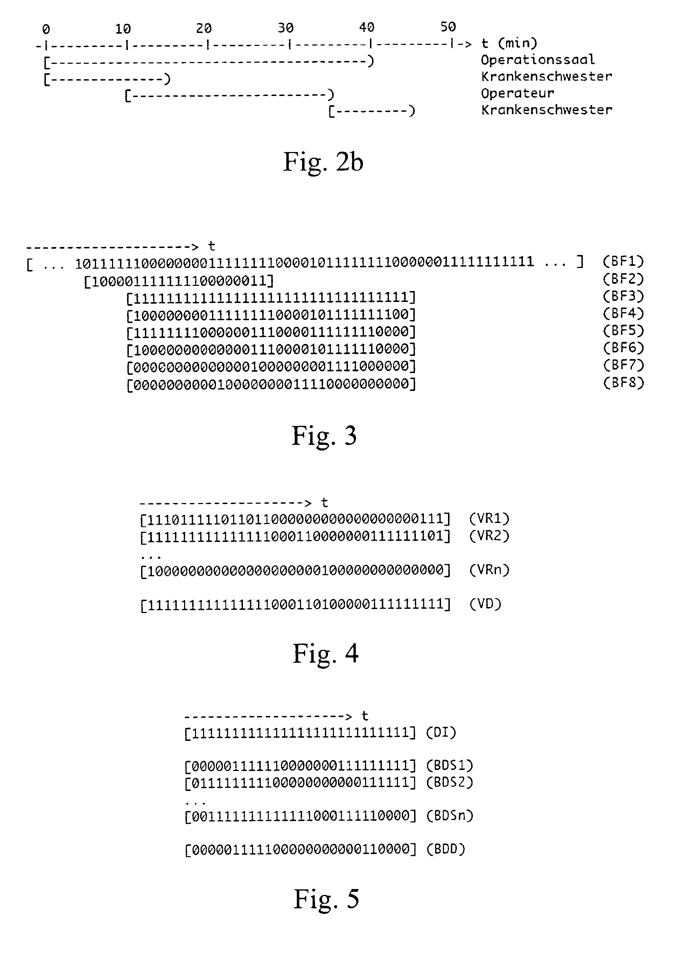

Die Figur 2b zeigt eine graphische Darstellung der in Figur 2a definierten Dienstleistungsart. Es werden die vier Dienstleistungsschritte und der Zeitraum dargestellt, in dem die Dienstleistungsschritte jeweils relativ zum Beginn der Dienstleistung insgesamt ablaufen und entsprechende Ressourcen benötigt werden. So wird ein Operationssaal vom Beginn der Dienstleistung bis einschließlich zur 40. Minute benötigt. Eine Krankenschwester wird vom Beginn der Dienstleistung bis einschließlich zur 15. Minute benötigt. Ein Operateur wird ab der 10. Minute der Dienstleistung bis einschließlich zur 35. Minute benötigt. Eine Krankenschwester wird ab der 35. Minute der Dienstleistung bis einschließlich zur 45. Minute benötigt.

Die Figur 3 zeigt, wie die Buchbarkeit einer Ressource innerhalb eines angefragten Zeitintervalls (BF3) berechnet werden kann, die für einen Dienstleistungsschritt einer Dauer (LNG) von 3 Minuten benötigt wird, welcher mit einer Zeitversetzung (STARTOFFSET) von 4 Minuten relativ zum Beginn der Dienstleistung beginnt. Im Ausführungsbeispiel der Figur 3 sind die Informationen über die Verfügbarkeiten und die Informationen über Buchungen einer Ressource jeweils als ein Bitstring dargestellt, wobei jedes Bit einem Zeitraum von einer Minute entspricht.

In der Figur 3 stellt t den Verlauf der Zeitachse dar. BF 1 ist ein Bitstring, der die Buchung der Ressource darstellt. Ein Bitwert von 1 bedeutet, dass die Ressource für die durch das Bit repräsentierte Minute gebucht ist, während ein Bitwert von 0 bedeutet, dass die Ressource für diese Minute nicht gebucht ist. Ein solcher Buchungs-Bitstring wird für jede Ressource im System gespeichert. BF 2 ist ein Bitstring, der die Verfügbarkeit (allgemeine Buchbarkeit bzw. Öffnungszeit) der Ressource darstellt. Ein Bitwert von 1 bedeutet, dass die Ressource in der durch das Bit repräsentierten Minute verfügbar ist, während ein Bitwert von 0 bedeutet, dass die Ressource in dieser Minute nicht verfügbar ist. Ein solcher Verfügbarkeits-Bitstring wird für jede Ressource im System gespeichert. BF3 ist ein Bitstring, der das angefragte Zeitintervall darstellt. In Figur 3 haben alle Bits von BF3 den Wert 1, was bedeutet, sich die Anfrage auf jede Minute in dem Zeitintervall bezieht, d.h. das elektronische Buchungssystem soll für jede Minute in dem Intervall prüfen, ob die Ressource buchbar ist, wenn die Dienstleistung in dieser Minute beginnt. BF4 ist ein Bitstring, der die Buchung der Ressource im angefragten Zeitintervall darstellt. Der Bitstring BF4 wird dadurch berechnet, dass die Bitstrings BF1 und BF3 durch eine logische UND-Operation bitweise verknüpft werden. BF5 ist ein Bitstring, der die Verfügbarkeit der Ressource im angefragten Zeitintervall darstellt. Da sich die Verfügbarkeiten (Öffnungszeiten) periodisch wiederholen, wird BF5 dadurch berechnet, dass jedes Bit des Strings, der das angefragte Zeitintervall darstellt, so auf die Verfügbarkeit abgebildet wird, dass gilt:

$$\text{OFFSET} = (\text{STARTINDEX(BF3)} - \text{STARTINDEX(BF2)}) \text{ MODULO LÄNGE(BF2)}$$

$$\text{BF5[i]} = \text{BF2[I + OFFSET) MODULO LÄNGE(BF2)]}$$

BF6 ist ein Bitstring, der eine Kombination aus Verfügbarkeit und Buchung der Ressource im angefragten Zeitraum darstellt. BF6 wird dadurch berechnet, dass die Bitstrings BF4 und BF5 durch eine logische UND-Operation bitweise verknüpft werden. BF7 ist ein Bitstring, der darstellt, zu welchen Startzeitpunkten die Ressource für die volle Dauer des Dienstleistungsschritts von 3 Minuten buchbar ist. BF7 wird dadurch berechnet, dass von allen aufeinander folgenden gesetzten Bits (Bits mit Wert 1) die letzten LNG - 1 (in Figur 3: 3 - 1 = 2) Bits gelöscht (auf den Wert 0) gesetzt werden. BF8 ist ein Bitstring, der die Buchbarkeit der Ressource für die volle Dauer des Dienstleistungsschritts unter Berücksichtigung der Zeitversetzung (STARTOFFSET) von 4 Minuten darstellt. BF8 wird dadurch berechnet, dass der Bitstring BF7 um STARTOFFSET Bits (in Figur 3: 4 Bits) nach links verschoben wird, was einer Multiplikation mit $2^{\text{STARTOFFSET}}$ entspricht.

In dem Fall, dass eine bestimmte Ressource eines Dienstleisters in einem Dienstleistungsschritt benötigt wird und es für diese Ressource keine Alternativen gibt, entspricht der in Figur 3 dargestellte Bitstring BF8, welcher die Buchbarkeit der bestimmten Ressource darstellt, auch der Buchbarkeit des Dienstleistungsschritts. Dagegen zeigt die Figur 4, wie die Buchbarkeit eines Dienstleistungsschritts für den Fall berechnet wird, dass das Buchungssystem mehrere (n) alternative Ressourcen ermittelt hat, von denen jede den Bedarf des Dienstleistungsschritts abdecken kann, etwa weil all diese Ressourcen eine für den Dienstleistungsschritt spezifizierte Fähigkeit besitzen.

In der Figur 4 stellt t den Verlauf der Zeitachse dar. VR1 bis VRn sind Bitstrings, die die Buchbarkeit der Ressourcen 1 bis n für den Dienstleistungsschritt in einem angegebenen Intervall darstellen. VR1 bis VRn werden so berechnet, wie oben in Bezug auf die Figur 3 beschrieben wurde. VD ist ein Bitstring, der für jede Minute des angegebenen Intervalls angibt, ob mindestens eine der mehreren alternativen Ressourcen buchbar ist. VD wird dadurch berechnet, dass die Bitstrings VR1 bis VRn durch eine logische ODER-Operation bitweise verknüpft werden.

Die Figur 5 zeigt, wie die Buchbarkeit einer Dienstleistung mit n Dienstleistungsschritten innerhalb eines angegebenen Zeitintervalls berechnet werden kann. In der Figur 5 stellt t wiederum den Verlauf der Zeitachse dar. BDS 1 bis BDSn sind Bitstrings, die die Buchbarkeit der Dienstleistungsschritte 1 bis n im angegebenen Intervall darstellen. BDS 1 bis BDSn werden so berechnet, wie oben in Bezug auf die Figuren 3 und 4 beschrieben wurde. BDD ist ein Bitstring, der für jede Minute des angegebenen Intervalls angibt, ob alle n Dienstleistungsschritte buchbar sind. BDD wird dadurch berechnet, dass die Bitstrings BDS1 bis BDSn durch eine logische UND-Operation bitweise verknüpft werden.

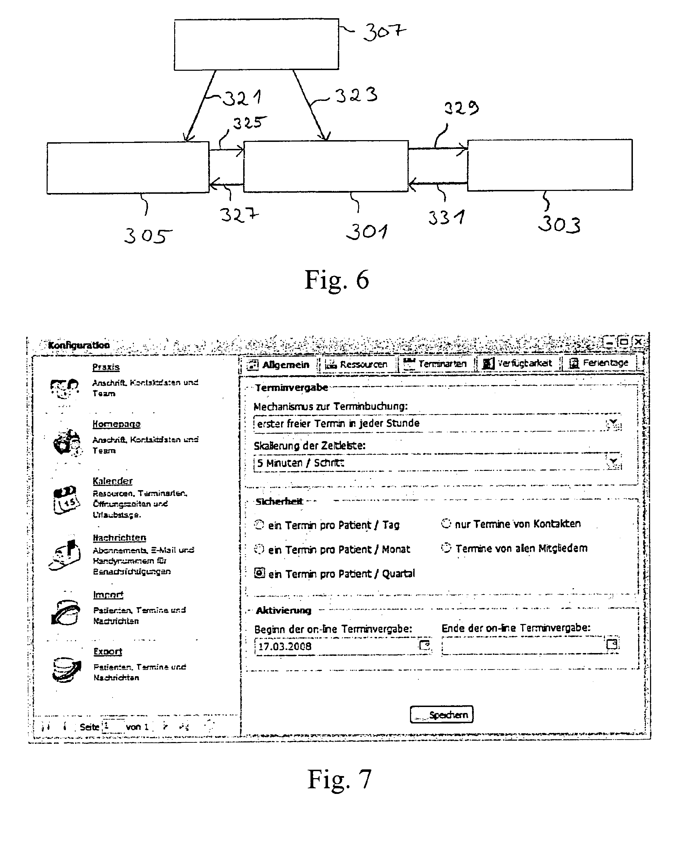

In einer Ausführungsform wird die Erfindung zur Terminvergabe für Patienten bei Arztpraxen und Krankenhäusern eingesetzt. Die Erfindung kann zum Beispiel im Rahmen eines Online-Terminbuchungssystems für eine automatische Online-Terminvergabe, eigene Praxisorganisation und interne Ressourcen-Optimierung verwendet werden, welches im Folgenden mit Bezug auf die Figuren 6 bis 9 beschrieben wird.

Ein integriertes Terminbuchungssystem kann die bestehenden Offline-Terminkalender vollständig ersetzen. Es wird kein manueller Abgleich zwischen Offline- und Online-Terminkalendern benötigt, da sowohl die klassische Terminvergabe, wie z.B. über Telefon oder Vorort in der Praxis, als auch die Online-Terminvergabe innerhalb eines Systems abläuft. Darüber hinaus bietet diese Ausführungsform jeder Praxis die Möglichkeit, nicht nur den Terminkalender sondern auch die Praxisabläufe individuell selbst zu konfigurieren. Ein solches Online-Terminbuchungssystem ist komfortabel vom Patienten zu bedienen (trotz unterschiedlicher Arztpraxen, Praxisgrößen und Patientenpräferenzen), und führt zu sehr effizienten, komplexen internen Abwicklungsmechanismen in den Praxen und Kliniken.

Fig. 6 zeigt eine schematische Darstellung der Struktur eines Internet-gestützten Systems zur Online-Terminbuchung. Das System zur Online-Terminbuchung beinhaltet dabei vier Ebenen der Bedienung und Steuerung: 1. Konfiguration 307, 2. Patient 305, 3. serverseitige Buchungsautomatik 301, 4. Praxis 303. Die Konfiguration 307 wechselwirkt mit dem Patienten bzw. Patienten-Rechner 305, der serverseitigen Buchungsautomatik 301 und der Praxis bzw. dem Praxis-Rechner 303. Die Konfiguration 307, in der die Praxiseigenschaften abgelegt sind, gibt dem Patienten Auswahloptionen vor, 321, und setzt der Buchungsautomatik 301, die durch IT-Algorithmen realisiert ist, Bedingungen, 323. Der Patient 305 nimmt bei der Buchungsautomatik 301 eine Terminbuchung vor, 325, nachdem ihm die Buchungsautomatik 301 wenigstens eine Termininformation geliefert hat, 327. Die Buchungsautomatik 301 nimmt eine Optimierung des Dienstplans und der Ressourcen vor, 329, und sendet diese, 331, an die Praxis 303. Von der Praxis aus können aufgrund höherer Benutzerrechte manuell Einträge im Terminkalender vorgenommen werden, die von der Buchungsautomatik abweichen.

Auf der ersten Ebene befindet sich die einmalige, manuelle online Konfiguration, mit der die Arztpraxen die Eigenschaften und Bedingungen festlegen, die die Terminauswahl für die Patienten und die Buchungsautomatik bestimmen. Damit können Praxen von einem Arzt bis zu einem ganzen Ärzte- und Personalteam konfiguriert werden. Hierfür kann ein Konfigurationsmenü entwickelt werden, das auf alle Praxisgrößen und -konditionen angepasst werden kann. Das Online-Konfigurationsmenü kann dabei zum Beispiel die folgenden neun Einstellungen umfassen:

1. Ressourcen: Stellen die buchbaren Personen oder Dinge einer Praxis dar, deren zeitliche Auslastung kritisch für einen effizienten Praxisablauf ist. Ressourcen können bspw. Ärzte, Assistentinnen, Geräte und/oder Räume sein, die zur Durchführung der einzelnen Termine benötigt werden. Für die Ressource wird die zeitliche Verfügbarkeit (Wochentage, Uhrzeiten) bestimmt sowie der Dienst, den sie anbietet.

2. Ferientage: legen die Abwesenheitstage für die personellen Ressourcen in der Praxis fest.

3. Einfache Terminarten: Bezeichnung (als Auswahl für den Patienten, wie z.B. Konsultation, Akupunktur), Beschreibung (als zusätzliche Information für den Patienten), zeitliche Dauer für eine Praxis-Ressource und farbliche Anzeige. Außerdem kann bestimmt werden, welche Patientengruppen diesen Termin buchen dürfen.

4. Terminblocker: Beinhalten die gleiche Funktionalität wie die ‚Einfache Terminart', bis auf die Ausnahme, dass der Termin nur intern von der Praxis gebucht werden kann und nicht extern von Patienten.

5. Komplexere Terminarten: Beinhalten die gleiche Funktionalität wie die 'Einfache Terminart', bis auf die Änderung, dass die Terminart mehrere Dienste von Ressourcen (also Ärzte, Assistenten, Räume oder Geräte buchen kann) beansprucht. Z.B. kann bei der Terminart Akupunktur festgelegt werden, dass der Termin insgesamt 30 Minuten dauert, einer von mehreren Räumen (Dienst: Verfügbarkeit einer Liege) für Akupunktur auch insgesamt 30 Minuten belegt wird, der Arzt als Ressource jedoch nur die ersten 5 Minuten benötigt wird und der Assistenzdienst (eine der Assistenz- Ressourcen) innerhalb der letzten 5 Minuten beansprucht wird.

6. Terminverfügbarkeit: Bestimmt, wann die Terminarten für jede Ressource verfügbar sind (d.h. die Wochentage und Uhrzeiten des Terminangebots).

7. Buchungsmechanismus: ermittelt das Angebot der freien Termine für die Patienten an einem bestimmten Tag, d.h. wann freie Termine online angeboten werden. Dabei stehen dabei dem Arzt zum Beispiel vier Optionen zur Auswahl:

(i) Dem Patienten werden alle freien Termine an dem gewählten Tag angeboten. Somit hat der Patient die freie Auswahl und die größte Flexibilität.

(ii) Dem Patienten wird nur der erste freie Termin in jeder Stunde angezeigt. Damit wird versucht, jede Stunde der Praxisressourcen systematisch zu füllen, was zu einer gleichmäßigen Praxisauslastung ohne Pause führt.

(iii) Die Praxis kann eine einfache Terminclusterung vorgeben, indem dem ersten Patienten alle Termine angeboten werden, aber die folgenden Patienten nur die genau vorhergehenden und genau nachfolgenden Termine auswählen können. Als Folge bleiben die gebuchten Termine in einem zusammenhängenden zeitlichen Block.

(iv) Eine multiple Terminclusterung läuft ähnlich wie die einfache Terminclusterung, nur dass die folgenden Termine nicht um den ersten Termin gruppiert werden, sondern um mehrere Zentren (wie bspw. den Starttermin, einen Mittagstermin und den Endtermin). Somit wird versucht, dem Patienten eine gewisse Flexibilität zu gewähren, aber trotzdem eine zusammenhängende Auslastung der Praxisressourcen zu erreichen.

8. Sicherheitseinstellungen: legen fest, wie oft ein Termin von einem Patienten online gebucht werden darf und ob nur ihm bekannte Patienten (aus seinem Kontaktverzeichnis) oder alle Patienten Termin bei ihm online buchen dürfen.

9. Aktivierung: bestimmt, wann das Online-Terminbuchungssystem anfangen und enden soll.

Fig. 7 zeigt ein Beispiel eines Konfigurationsmenüs zur Konfiguration des Systems nach Fig. 6. Auf der vierten Ebene (Praxis) befindet sich die operative Verwaltungsansicht für die Praxis in Form einer klassischen Terminkalenderansicht, wie in Fig. 7 gezeigt. Diese Ansicht kann hinsichtlich Farbgestaltung etc. durch die Praxis in der Software konfiguriert werden.

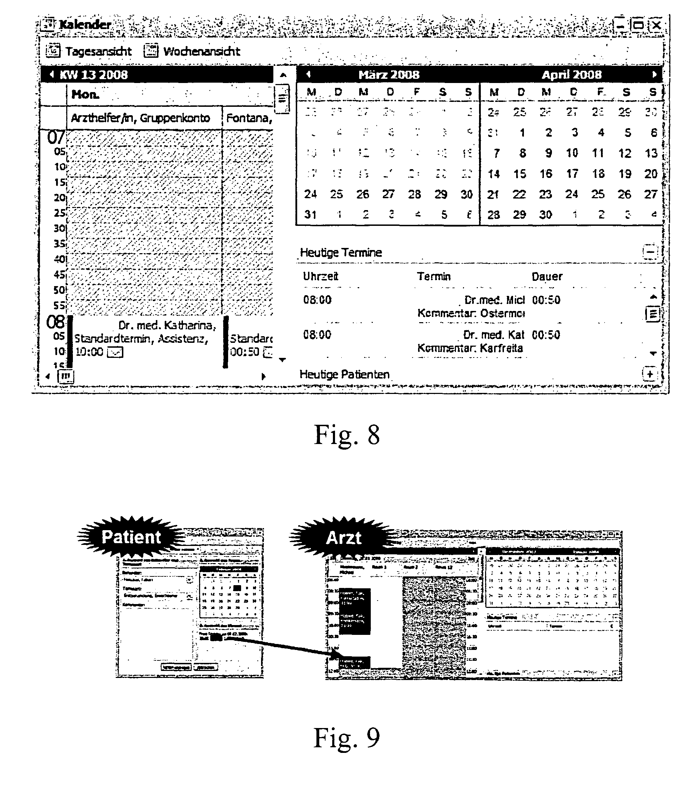

Fig. 8 zeigt ein Beispiel einer Terminkalenderansicht aus der Sicht der Praxis. In dieser Ansicht können die Praxismitarbeiter durch Anklicken auf die Kalenderspalte einen Termin eintragen. Der Termineintrag läuft ähnlich wie der des Patienten ab, nur dass die Konfigurationsvorgaben nicht zwingend sind. In einem erweiterten Termineingabeformular werden die Vorgaben explizit aufgeführt und können von den Praxismitarbeitern manuell abgeändert werden.

Auf der zweiten Ebene (Patient) befindet sich die Terminbuchungsansicht für die externen Benutzer, also die Patienten. Dank der vorangegangenen Konfiguration können die externen Benutzer jederzeit über Internet einen Termin buchen. Konkret heißt dies für die Erfindung, dass Patienten 24 Stunden, 7-Tage die Woche unabhängig von Feiertagen und Praxisöffnungszeiten einen Termin bei Ihren Ärzten (die der Software angeschlossen sind) vereinbaren können. Um die Termine buchen zu können, eröffnen Patienten beim Server ein Patienten-Benutzerkonto. Trotz der Komplexität unterschiedlicher Praxen und der Vielzahl von unterschiedlichen Terminkombinationen können die Ärzte mit dem Konfigurationsmenü ihren Patienten eine sehr einfache Online-Terminvergabe ermöglichen: Der Patient kann mit Hilfe des Servers in nur drei Schritten online einen Arzttermin buchen.

1. Zuerst wählt der Patient den Arzt aus seinem Kontaktverzeichnis aus.

2. Danach wählt er die Terminart aus, die der Arzt in der Konfiguration angegeben hat und den Patienten online zur Verfügung stellt. Dabei kann der Patient sich noch durch die entsprechenden Terminbeschreibungen informieren bzw. aufklären lassen.

3. Als letztes wählt der Patient den gewünschten Tag aus.

Anschließend ermitteln die erfindungsgemäßen Buchungsalgorithmen eine Auswahl von freien Terminen. Der Patient kann sich daraus seine Wunschtermine aussuchen und bestätigen, so dass der Termin gebucht und beim Arzt angezeigt wird, siehe Fig. 9.

In einem Notizfeld kann der Patient zusätzlich noch Fragen oder Anmerkungen zum Termin einstellen. Der Kommentar wird im Terminkalender angezeigt und nach der Beantwortung bzw. zur Kenntnisnahme als bearbeitet gekennzeichnet. Innerhalb der Konfiguration kann auch eingestellt werden, dass online gebuchte Termine nur nach Bestätigung durch die Praxis in den Kalender übernommen werden. Abschließend erhält der Patient eine Terminbestätigungs-Mail. Zusätzlich wird der Patient, sofern die Praxis dies konfiguriert hat und der Patient dies in seinem persönlichen Benutzerkonto akzeptiert hat, XX Stunden vor dem Termin per SMS oder Mail an den Termin erinnert.

Im Terminkalender ist zusätzlich noch eine Terminverschiebungsmöglichkeit eingebaut, sofern das Wartezimmer voll ist, oder z.B. ein Notfall in der Praxis dazwischen gekommen ist, kann die Praxis die Patienten per SMS (sofern der jeweilige Patient dem in seinem persönlichen Benutzerkonto zugestimmt hat) über die längeren Wartezeiten informiert.

Auf der dritten Ebene (serverseitige erfindungsgemäße Buchungsautomatik) befindet sich die Steuerung der Buchungsautomatik. Die zugrundeliegenden Algorithmen können auf Basis eines Ruby-/JavaScript-Frameworks programmiert werden.

Durch die Anbindung der Ärzte an die Buchungssoftware ist es möglich, in der Arztsuche nicht nur nach klassischen Kriterien wie Fachgebiet, Name und Adressen, sondern auch nach Therapien, Geräten und Praxisphilosophien zu suchen.

Insbesondere durch die Anbindung der Ärzte an das Online-Terminbuchungssystem ist es möglich, Suche nach freien Terminen bei bestimmten Fachgebieten und Regionen durchzuführen, wie z.B. die Suche nach einem freien Termin bei einem Orthopäden in Berlin-Mitte heute ab 16 Uhr.

Der Praxis kann neben dem Terminkalender mit Online-Buchungssystem noch ein Praxis- bzw. Patientenmanagementtool zur Verfügung stehen, was ihr beispielsweise ermöglicht, die Patienten rechtzeitig an Termine zu erinnern, eine Recallsystematik für wieder anstehende Vorsorgeleistungen durchzuführen, Patienten in Gruppen zur besseren Verwaltung und Information einzuteilen.

**Patentansprüche**

1. Vorrichtung zur elektronischen Buchung einer Dienstleistung, umfassend:

Mittel zur Speicherung von Informationen über Dienstleistungsarten, die einem Dienstleister zugeordnet sind;
Mittel zur Speicherung von Informationen über Ressourcen, die dem Dienstleister zugeordnet sind;
Mittel zur Speicherung von Informationen über die Verfügbarkeiten, die einer Ressource zugeordnet sind;
Mittel zur Speicherung von Informationen über Buchungen, die einer Ressource zugeordnet sind;
Mittel zur Speicherung einer Liste von Informationen über Dienstleistungsschritte, die einer Dienstleistungsart zugeordnet sind, wobei Informationen über einen Dienstleistungsschritt Informationen über mindestens eine Ressource und Informationen über einen Zeitraum umfasst, in dem die Ressource benötigt wird;

eine Schnittstelle zum Erhalten einer Buchungsanfrage, die eine Dienstleistungsart spezifiziert; und

Mittel zur Berechnung von Zeiten, zu denen die in der Buchungsanfrage spezifizierte Dienstleistungsart beim Dienstleister gebucht werden kann, wobei die Mittel dazu eingerichtet sind, die Berechnung anhand der der spezifizierten Dienstleistungsart zugeordneten Liste von Informationen über Dienstleistungsschritte, der Informationen über Ressourcen, die dem Dienstleister zugeordnet sind, der Informationen über die Verfügbarkeiten, die den Ressourcen zugeordnet sind und der Informationen über Buchungen, die den Ressourcen zugeordnet sind, durchzuführen.

**2.** Vorrichtung nach Anspruch 1, wobei

die Vorrichtung Mittel zur Speicherung einer Liste von Informationen über Fähigkeiten, die einer Ressource zugeordnet sind umfasst;

Informationen über einen Dienstleistungsschritt die Informationen über mindestens eine Ressource als Informationen über eine benötigte Fähigkeit umfassen; und

die Mittel zur Berechnung von Zeiten, zu denen die in der Buchungsanfrage spezifizierte Dienstleistungsart beim Dienstleister gebucht werden kann, dazu eingerichtet sind, anhand der Informationen über die benötigte Fähigkeit Informationen über Ressourcen zu ermitteln, denen die benötigte Fähigkeit zugeordnet ist.

**3.** Vorrichtung nach Anspruch einem der vorhergehenden Ansprüche, wobei die Informationen über die Verfügbarkeiten einen Bitstring umfassen, in dem jedes Bit die Verfügbarkeit in einem bestimmten Zeitintervall beschreibt;

die Informationen über Buchungen einen Bitstring umfassen, aus dem ermittelt werden kann, zu welchem Zeitpunkt gebucht oder nicht gebucht ist;

und

die Mittel zur Berechnung von Zeiten, zu denen die in der Buchungsanfrage spezifizierte Dienstleistungsart beim Dienstleister gebucht werden kann, dazu eingerichtet sind, für einen gegebenen Zeitraum die Zeiten **dadurch** zu berechnen, dass:

die der spezifizierten Dienstleistungsart zugeordneten Dienstleistungsschritte ermittelt werden;

für die ermittelten Dienstleistungsschritte jeweils mindestens eine Ressource ermittelt wird;

für die ermittelten Ressourcen die Verfügbarkeiten und die Buchungen ermittelt werden, die der jeweiligen Ressource zugeordnet sind;

die Bitstrings der ermittelten Verfügbarkeiten und Buchungen durch mindestens eine erste Operation so verknüpft werden, dass jeweils ein Bitstring erzeugt wird, der die Buchbarkeit der jeweiligen Ressource im gegebenen Zeitraum beschreibt;

aus der Buchbarkeit der für einen Dienstleistungsschritt ermittelten Ressourcen ein Bitstring ermittelt wird, der die Buchbarkeit des jeweiligen Dienstleistungsschritts im gegebenen Zeitraum beschreibt;

die ermittelten Bitstrings der Buchbarkeiten der Dienstleistungsschritte durch mindestens eine zweite Operation so verknüpft werden, dass ein Bitstring erzeugt wird, der die Buchbarkeit der spezifizierten Dienstleistungsart im gegebenen Zeitraum beschreibt.

**4.** Vorrichtung nach Anspruch 3, wobei die Mittel zur Berechnung von Zeiten, zu denen die in der Buchungsanfrage spezifizierte Dienstleistungsart beim Dienstleister gebucht werden kann, dazu eingerichtet sind, zur Ermittlung des Bitstrings, der die Buchbarkeit eines Dienstleistungsschritts im gegebenen Zeitraum beschreibt, die erzeugten Bitstrings der Buchbarkeiten der für den Dienstleistungsschritt ermittelten Ressourcen durch mindestens eine dritte Operation so zu verknüpfen, dass der zu ermittelnde Bitstring erzeugt wird.

**5.** Vorrichtung nach einem der vorhergehenden Ansprüche, wobei die Vorrichtung eine Schnittstelle aufweist, über die Buchungsvorgaben in Form einer Basiskonfiguration des Dienstleisters empfangen werden können.

**6.** Vorrichtung nach einem der vorhergehenden Ansprüche, wobei die Vorrichtung eine Interaktionseinheit aufweist, die dazu eingerichtet ist, auf eine Buchungsanfrage hin eine Mehrzahl von Dienstleistern anzuzeigen, die die angefragte Dienstleistungsart bereitstellen.

**7.** Vorrichtung nach Anspruch 6, wobei die Interaktionseinheit dazu eingerichtet ist, die angezeigten Dienstleister nach ihrer räumlichen Entfernung zu einem Nutzer sortiert anzuzeigen.

**8.** Vorrichtung nach Anspruch 6, wobei die Interaktionseinheit dazu eingerichtet ist, die angezeigten Dienstleister nach der Dauer bis zum frühest möglichen Zeitpunkt der Erbringung der Dienstleistungsart sortiert anzuzeigen.

**9.** Verfahren zur elektronischen Buchung einer Dienstleistung, umfassend:

Speichern von Informationen über Dienstleistungsarten, die einem Dienstleister zugeordnet sind;

Speichern von Informationen über Ressourcen, die dem Dienstleister zugeordnet sind;

Speichern von Informationen über die Verfügbarkeiten, die einer Ressource zugeordnet sind;

Speichern von Informationen über Buchungen, die einer Ressource zugeordnet sind;

Speichern einer Liste von Informationen über Dienstleistungsschritte, die einer Dienstleistungsart zugeordnet sind, wobei Informationen über einen Dienstleistungsschritt Informationen über mindestens eine Ressource und Informationen über einen Zeitraum umfasst, in dem die Ressource benötigt wird;

Erhalten einer Buchungsanfrage, die eine Dienstleistungsart spezifiziert; und

Berechnen von Zeiten, zu denen die in der Buchungsanfrage spezifizierte Dienstleistungsart beim Dienstleister gebucht werden kann, anhand der der spezifizierten Dienstleistungsart zugeordneten Liste von Informationen über Dienstleistungsschritte, der Informationen über Ressourcen, die dem Dienstleister zugeordnet sind, der Informationen über die Verfügbarkeiten, die den Ressourcen zugeordnet sind und der Informationen über Buchungen, die den Ressourcen zugeordnet sind.

10. Verfahren nach Anspruch 9, wobei

das Verfahren Speichern einer Liste von Informationen über Fähigkeiten, die einer Ressource zugeordnet sind umfasst;

Informationen über einen Dienstleistungsschritt die Informationen über mindestens eine Ressource als Informationen über eine benötigte Fähigkeit umfassen; und

zur Berechnung von Zeiten, zu denen die in der Buchungsanfrage spezifizierte Dienstleistungsart beim Dienstleister gebucht werden kann, anhand der Informationen über die benötigte Fähigkeit Informationen über Ressourcen ermittelt werden, denen die benötigte Fähigkeit zugeordnet ist.

11. Verfahren nach einem der Ansprüche 9 bis 10, wobei

die Informationen über die Verfügbarkeiten einen Bitstring umfassen, in dem jedes Bit die Verfügbarkeit in einem bestimmten Zeitintervall beschreibt;

die Informationen über Buchungen einen Bitstring umfassen, aus dem ermittelt werden kann, zu welchem Zeitpunkt gebucht oder nicht gebucht ist; und

zur Berechnung von Zeiten, zu denen die in der Buchungsanfrage spezifizierte Dienstleistungsart beim Dienstleister gebucht werden kann, für einen gegebenen Zeitraum die Zeiten **dadurch** berechnet werden, dass:

die der spezifizierten Dienstleistungsart zugeordneten Dienstleistungsschritte ermittelt werden;

für die ermittelten Dienstleistungsschritte jeweils mindestens eine Ressource ermittelt wird;

für die ermittelten Ressourcen die Verfügbarkeiten und die Buchungen ermittelt werden, die der jeweiligen Ressource zugeordnet sind;

die Bitstrings der ermittelten Verfügbarkeiten und Buchungen durch mindestens eine erste Operation so verknüpft werden, dass jeweils ein Bitstring erzeugt wird, der die Buchbarkeit der jeweiligen Ressource im gegebenen Zeitraum beschreibt;

aus der Buchbarkeit der für einen Dienstleistungsschritt ermittelten Ressourcen ein Bitstring ermittelt wird, der die Buchbarkeit des jeweiligen Dienstleistungsschritts im gegebenen Zeitraum beschreibt;

die ermittelten Bitstrings der Buchbarkeiten der Dienstleistungsschritte durch mindestens eine zweite Operation so verknüpft werden, dass ein Bitstring erzeugt wird, der die Buchbarkeit der spezifizierten Dienstleistungsart im gegebenen Zeitraum beschreibt.

12. Verfahren nach Anspruch 11, wobei zur Berechnung von Zeiten, zu denen die in der Buchungsanfrage spezifizierte Dienstleistungsart beim Dienstleister gebucht werden kann, zur Ermittlung des Bitstrings, der die Buchbarkeit eines Dienstleistungsschritts im gegebenen Zeitraum beschreibt, die erzeugten Bitstrings der Buchbarkeiten der für den Dienstleistungsschritt ermittelten Ressourcen durch mindestens eine dritte Operation so verknüpft werden, dass der zu ermittelnde Bitstring erzeugt wird.

13. Verfahren nach einem der Ansprüche 9 bis 12, wobei Buchungsvorgaben in Form einer Basiskonfiguration des Dienstleisters empfangen werden.

14. Verfahren nach einem der Ansprüche 9 bis 13, wobei auf eine Buchungsanfrage hin eine Mehrzahl von Dienstleistern angezeigt wird, die die angefragte Dienstleistungsart bereitstellen.

15. Computerprogramm, das, wenn es auf einem oder mehreren Computern ausgeführt wird bewirkt, dass das Verfahren nach einem der Ansprüche 9 bis 14 durchgeführt wird.

Fig. 1

```
* Dienstleistung:
  * Name: 'Operation'
  * Dienstleistungsschritt:
      * Name: 'Operationssaal'
      * FÄHIGKEIT: 'Operationssaal'
      * LNG: 40 Minuten
      * STARTOFFSET: 0 Minuten
  * Dienstleistungsschritt:
      * Name: 'Vorbereitung'
      * FÄHIGKEIT: 'Krankenschwester'
      * LNG: 15 Minuten
      * STARTOFFSET: 0 Minuten
  * Dienstleistungsschritt:
      * Name: 'Operation durchführen'
      * FÄHIGKEIT: 'Operateur'
      * LNG: 25 Minuten
      * STARTOFFSET: 10 Minuten
  * Dienstleistungsschritt:
      * Name: 'Nachbereitung'
      * FÄHIGKEIT: 'Krankenschwester'
      * LNG: 10 Minuten
      * STARTOFFSET: 35 Minuten
```

Fig. 2a

```
    0         10        20        30        40        50
 -|---------|---------|---------|---------|---------|-> t (min)
  [----------------------------------------)         Operationssaal
  [--------------)                                    Krankenschwester
            [------------------------)                Operateur
                                      [--------)      Krankenschwester
```

## Fig. 2b

```
--------------------> t
[ ... 1011111100000000011111111100000101111111100000011111111111 ... ]  (BF1)
         [10000011111111100000011]                                       (BF2)
               [1111111111111111111111111111111111]                      (BF3)
               [10000000001111111110000101111111100]                     (BF4)
               [1111111100000011100001111111110000]                      (BF5)
               [1000000000000001110000101111110000]                      (BF6)
               [0000000000000001000000001111000000]                      (BF7)
               [0000000000010000000001111000000000]                      (BF8)
```

## Fig. 3

```
--------------------> t
[1110111110110110000000000000000000111]   (VR1)
[11111111111111110001100000000111111101]  (VR2)
...
[10000000000000000000001000000000000000]  (VRn)

[11111111111111110001101000001111111111]  (VD)
```

## Fig. 4

```
--------------------> t
[11111111111111111111111111111]  (DI)

[000001111110000000011111111]    (BDS1)
[011111111100000000000111111]    (BDS2)
...
[00111111111111110001111110000]  (BDSn)

[000001111100000000000110000]    (BDD)
```

## Fig. 5

Fig. 6

Fig. 7

Fig. 8

Fig. 9

Europäisches
Patentamt

European
Patent Office

Office européen
des brevets

**EUROPÄISCHER RECHERCHENBERICHT**

Nummer der Anmeldung

EP 09 00 5546

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2005/004815 A1 (MACHTELINCK GEERT [BE]) 6. Januar 2005 (2005-01-06) * Zusammenfassung; Ansprüche 1-24; Abbildungen 1-4 * * Absätze [0020], [0024], [0025], [0030] - [0033], [0037] - [0044], [0049] - [0051], [0056] - [0062] * | 1-15 | INV. G06F19/00 |
| X | US 2006/143060 A1 (CONRY ANNE M [US] ET AL) 29. Juni 2006 (2006-06-29) * Zusammenfassung; Ansprüche 1-10; Abbildungen 1-4 * * Seite 1 - Seite 8 * | 1-15 | |
| X | US 2005/228696 A1 (EGAWA HIROSHI [JP]) 13. Oktober 2005 (2005-10-13) * Zusammenfassung; Ansprüche 1-18; Abbildungen 1-9 * * Seiten 2-7 * | 1-15 | |
| A | WO 2006/094890 A (QUADRAT [BE]; MACHTELINCK GEERT [BE]) 14. September 2006 (2006-09-14) * Zusammenfassung; Ansprüche 1-10 * * Seiten 6-7 * * Seiten 16-19 * | 1-15 | RECHERCHIERTE SACHGEBIETE (IPC) G06F G06Q |
| A | "BIT VECTOR FOR OPTIMAL PERFORMANCE OF CALENDAR BAR CONTROL" IBM TECHNICAL DISCLOSURE BULLETIN, IBM CORP. NEW YORK, US, Bd. 36, Nr. 10, 1. Oktober 1993 (1993-10-01), Seiten 621-623, XP000412508 ISSN: 0018-8689 * das ganze Dokument * | 1-15 | |

-/--

Der vorliegende Recherchenbericht wurde für alle Patentansprüche erstellt

| Recherchenort | Abschlußdatum der Recherche | Prüfer |
|---|---|---|
| Den Haag | 23. Juli 2009 | Streit, Stefan |

Europäisches
Patentamt

European
Patent Office

Office européen
des brevets

# EUROPÄISCHER RECHERCHENBERICHT

Nummer der Anmeldung

EP 09 00 5546

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| P,X | DE 20 2008 007826 U1 (SAMEDI GMBH [DE]) 28. August 2008 (2008-08-28) * das ganze Dokument * ----- | 1-15 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| Den Haag | 23. Juli 2009 | Streit, Stefan |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
.................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 09 00 5546

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-07-2009

| Im Recherchenbericht angeführtes Patentdokument | Datum der Veröffentlichung | Mitglied(er) der Patentfamilie | Datum der Veröffentlichung |
|---|---|---|---|
| US 2005004815 A1 | 06-01-2005 | CN 1826611 A<br>WO 2005004013 A1<br>JP 2007527042 T | 30-08-2006<br>13-01-2005<br>20-09-2007 |
| US 2006143060 A1 | 29-06-2006 | KEINE | |
| US 2005228696 A1 | 13-10-2005 | AU 2002328597 A1<br>WO 2004015605 A1 | 25-02-2004<br>19-02-2004 |
| WO 2006094890 A | 14-09-2006 | KEINE | |
| DE 202008007826 U1 | 28-08-2008 | KEINE | |

EPO FORM P0461

Für nähere Einzelheiten zu diesem Anhang : siehe Amtsblatt des Europäischen Patentamts, Nr.12/82